# EUROPÄISCHE PATENTSCHRIFT

(11) **EP 3 536 293 B1**
(45) Veröffentlichungstag und Bekanntmachung des Hinweises auf die Patenterteilung: **27.10.2021**
(21) Anmeldenummer: 18160687.2
(22) Anmeldetag: 08.03.2018
(51) Int. Cl.: A61F 13/42, G01N 27/12

(54) **SYSTEM ZUR ÜBERWACHUNG DES BELADUNGSZUSTANDS VON SAUGFÄHIGEN HYGIENEARTIKELN UND ZUR AUSWERTUNG DER DABEI GEWONNENEN MESSWERTE**
SYSTEM FOR MONITORING THE LOADING STATE OF ABSORBENT HYGIENE ARTICLES AND FOR EVALUATING MEASUREMENTS OBTAINED THEREBY
SYSTÈME DE SURVEILLANCE DE L'ÉTAT DE CHARGEMENT DES ARTICLES D'HYGIÈNE ABSORBANTS ET D'ÉVALUATION DE LA VALEUR MESURÉE AINSI OBTENUE

(43) Veröffentlichungstag der Anmeldung: 11.09.2019
(73) Patentinhaber: Curaluna GmbH, 60437 Frankfurt am Main (DE)
(72) Erfinder: STEINMETZ, Frank, 60437 Frankfurt am Main (DE)
(74) Vertreter: K&P Patentanwaltsgesellschaft mbH

(56) Entgegenhaltungen:
- WO-A1-2008/038166
- WO-A1-2011/030114
- WO-A1-2013/013197
- WO-A1-2014/122169
- US-A1- 2004 220 538
- US-A1- 2016 095 758
- US-A1- 2017 162 931
- US-A1- 2017 354 374
- US-B1- 9 675 496

## Beschreibung

Die Erfindung betrifft ein System zur Überwachung des Beladungszustands von saugfähigen Hygieneartikeln und zur Auswertung der dabei gewonnenen Messwerte.

Ein System zur Überwachung von Inkontinenz ist aus der US 2017/0354374 A1 bekannt. Ein dort verwendeter Sensor benötigt zur Detektierung einer Feuchtigkeit eine in Wasser lösbare, elektrisch leitfähige Substanz. Dadurch ist der Sensor nach einmaliger Verwendung verbraucht und ist nicht wiederverwendbar.

Ein weiteres System zur Überwachung von Inkontinenz ist aus der DE 32 23 036 A1 bekannt. In einem oder an einem saugfähigen Hygieneartikel wird eine separate Elektrodeneinlage für eine ohmsche Widerstandsmessung eingearbeitet oder angeordnet, die über eine lösbare elektrische Leitung mit einem Mess- und Sendeteil verbunden ist. Das Mess- und Sendeteil wird von dem den Hygieneartikel verwendenden Benutzer mitgeführt. Es steht seinerseits mit einem Empfänger in Verbindung, über welchem einem beaufsichtigenden Personal ein akustisches oder optisches Signal infolge einer vom Befeuchtungsgrad des Hygieneartikels abhängigen Widerstandsänderung übermittelt wird.

Auch aus der WO 2013 013 197 A1, der WO 2014 122 169 A1, der US 2017 162 931 A1, der US 2004 220 538 A1 und der WO 2011 030 114 A1 sind Systeme zum Detektieren und Überwachen von Inkontinenz mittels unterschiedlicher Sensoren bekannt. Beispielsweise beschreibt die WO 2013 013 197 A1 einen Flüssigkeitssensor mit einer galvanisch erregbaren Stromquelle, die in der Lage ist, ein detektierbares Signal als Reaktion auf eine Flüssigkeit zu erzeugen.

Zurzeit gibt es verschiedene Systeme am Markt, welche verschiedene Ziele verfolgen. So gibt es zum einen Alarmsysteme, welche das Abgewöhnen von Enuresis (Bettnässen) erleichtern sollen. Diese besitzen einen Sensor, welcher Nässe erkennt und geben die Signale per Kabel oder Funk an einen Alarmgeber weiter, welcher den Betroffenen weckt. Dies soll dazu führen, dass der Betroffene nach einiger Trainingszeit von alleine aufwacht und die Toilette aufsuchen kann. Als Beispiele kann man den "Enuresis-Alarm" der chinesischen Firma F-Star nennen, oder das kabellose System "DRI-Eclipse" von Anzacare.

Des Weiteren gibt es Systeme, welche das Wasserlassen statistisch erfassen sollen, um die Toiletten- und Wickelzeiten anzupassen und im Voraus zu planen. Als Beispiel für ein System dieser Art wäre von Tena das System "Tena-Identifi" zu nennen.

Weiterhin gibt es bereits verschiedene Systeme, welche dem Anwender signalisieren sollen, dass eine getragene Windel gewechselt werden muss. Diese Systeme senden meist eine Benachrichtigung auf eine Smartphone-App. Beispielhaft wären hier "DDiaper" von Shenzen Hyan Microelectronics und "Diaper Alert" von One2Baby zu nennen. Diese Systeme nutzen verschiedene Sensorik; so nutzt beispielsweise "DDiaper" einen Nässesensor, welcher auf Nässe reagiert, nicht aber den Feuchtigkeitsgehalt angeben kann. "Diaper Alert" nutzt Feuchtigkeitssensoren, welche sowohl den Feuchtigkeitsgehalt als auch die Temperatur angeben können und somit um ein Vielfaches genauer sind. Aus der US 9 820 891 B2 ist ein optisches Verfahren bekannt, das in Kombination mit kapazitiven Sensoren den Beladungszustand einer Windel ermittelt.

Weiterhin nutzen die bekannten Systeme verschiedene Wege der Datenübertragung. Beispielsweise nutzt "Diaper Alert" Bluetooth mit geringer Reichweite. Auch SMS (IBN-Wet Alert) kommt zum Einsatz.

Die meisten der genannten Alarmsysteme wenden sich nur an Anwender, welche selber in der Lage sind, die Toilette zu erreichen. Sie erleichtern also weder Eltern noch Pflegern die Aufgabe des rechtzeitigen Windelwechselns. Die statistischen Systeme richten sich zwar an die Gruppe der Senioren, allerdings sind dies keine dauerhaften Anwendungen, sondern erfassen nur über einen relativ kurzen Zeitraum Daten und dienen dem zukünftigen Planen von Toilettenzeiten. Eine schnelle Anpassung bei geänderten Gewohnheiten oder Lebensumständen ist nicht ohne erneute Datenerfassung möglich.

Die Systeme, welche den Zeitpunkt zum Wechseln der Windeln anzeigen, wenden sich bislang nur an Einzelanwender, was den Einsatz in Pflegeeinrichtungen schwierig macht. Außerdem sind sie meist auf die Zielgruppe der Babys gerichtet, was sich auch in der Optik der Smartphone-Anwendungen widerspiegelt. Außerdem wird bei bekannten Systemen bislang keine Unterscheidung zwischen Stuhlgang und Urin getroffen. Die verwendeten Nässesensoren können nur das Vorhandensein elektrisch-leitender Nässe anzeigen, sie kennen also nur "nass" oder "trocken" und sind somit sehr ungenau. Außerdem können sie meist nur auf kleiner Fläche messen, was die Gefahr von Messfehlern erhöht.

Die Übertragungsart der bekannten Systeme ist mit großen Nachteilen behaftet. Kabelgebundene Systeme besitzen keinerlei Reichweite und sind unbequem zu tragen. Bei den Systemen mit Bluetooth besteht ein Problem mit der Reichweite, so beträgt diese beim bisher üblichen Bluetooth teilweise nur bis zu 10 Metern. Auch SMS werden zur Datenübertragung genutzt; diese sind aufgrund der Übertragungskosten kostspielig, sehr energieintensiv und nicht für eine Echtzeitüberprüfung anwendbar.

Weiterhin ist allen bisherigen Systemen gleich, dass diese keinerlei zentrale Verarbeitung gemessener Daten besitzen und somit auch nicht für weitere Verarbeitungen geeignet sind, wie beispielsweise prioritätsgesteuerten Einsatzplanung von Pflegepersonal, Ressourcen- oder Routenplanung für ambulante Pflegekräfte etc. zu unterstützen.

Aufgabe der Erfindung ist es, ein deutlich verbessertes, wiederverwendbares System zur Überwachung des Beladungszustands von saugfähigen Hygieneartikeln sowie ein Informationssystem für einen verbesserten Informationsfluss bereitzustellen.

Diese Aufgabe wird durch ein System mit den Merkmalen des Anspruchs 1 gelöst. Vorteilhafte Ausgestaltungen der Erfindung sind in den darauf bezogenen Unteransprüchen angegeben.

Mit der vorliegenden Erfindung ist eine exaktere Messung des Beladungszustands dadurch möglich, dass der am saugfähigen Hygieneartikeln anbringbare Sensor außer der Messung des Feuchtigkeitsverlaufs, also der Veränderung des Feuchtigkeitszustands über der Zeit, auch zur Messung der Temperatur des saugfähigen Hygieneartikels eingerichtet ist.

Das erfindungsgemäße System ist insbesondere vorteilhaft in Verbindung mit einer zentralen oder verteilten Datenbank verwendbar, aus der heraus handlungsrelevante Maßnahmen im Bereich pflegebedürftiger Menschen abgeleitet werden.

Ein zur Realisierung der Erfindung geeigneter Sensor ist z.B. der kombinierte Feuchtigkeits- und Temperatursensor "SI7021-A20" der Firma Silicon Labs. Die Anwendung des Sensors ist einfach gestaltet. Er wird erfindungsgemäß außen an der Windel befestigt und entweder per Bluetooth mit einer Smartphone-App oder per Übertragungsprotokoll mit dem NB-loT-Netzwerk verbunden. Von diesem Zeitpunkt an sendet er die Daten in Echtzeit zeit- oder ereignisgesteuert zur Auswertung an das Smartphone bzw. in der Verlängerung an die zentral oder verteilt vorgehaltene Datenbank, in der alle Messdaten zur weiteren Verarbeitung zusammenlaufen. Der beim erfindungsgemäßen System verwendete Sensor ist wiederverwendbar, d. h. er wird einfach von einem verbrauchten saugfähigen Artikel entfernt und an einem neuen saugfähigen Artikel angeordnet, beispielsweise mittels einer Klettverbindung, einer magnetischen Verbindung oder einer lösbaren Klebeverbindung.

Ein weiterer Nutzen der Erfindung liegt darin, dass die Datenübertragung zwischen dem unmittelbar mit dem Sensor verbundenen Sender und einem Empfänger kabellos in Echtzeit mit einer Reichweite erfolgt, die über der Reichweite einer einfachen Bluetooth®-Verbindung (SRD, Short Range Devices) von etwa 10 m hinausgeht. Hierbei werden in der Regel Reichweiten von deutlich mehr als 10 m und mindestens 200 m im Falle der Übertragungstechnik Bluetooth® 4.0 oder 5.0 (Long Range Module) erreicht. Im Falle von NB-loT liegt die theoretische Übertragungsreichweite bis zur nächsten Empfangsantenne bei bis zu 50 km.

Durch die kabellose Übertragung ist eine große Flexibilität bezüglich des Bewegungsspielraums eines mit einem saugfähigen Hygieneartikel versehenen Patienten gewährleistet. Die hohe Reichweite garantiert das Erkennen des Beladungszustands auch in größeren Einrichtungen, wie beispielsweise Krankenhäusern, Pflegestationen oder Altenheimen. Ein weiterer Vorteil der Erfindung liegt darin, dass der Empfänger das Signal an wenigstens eine als Frontend dienende, von einem Pflegepersonal einsehbare Anzeigevorrichtung übermittelt.

Besonders vorteilhaft ist die Anzeigevorrichtung für einen Empfang von Signalen mehrerer, an unterschiedlichen Orten oder Patienten angeordneten Hygieneartikeln geeignet und eingerichtet. Somit kann eine einzige Aufsichtsperson mittels einer einzigen Anzeigevorrichtung gleichzeitig eine Vielzahl von Patienten oder Nutzern derartiger Hygieneartikel überwachen, um rechtzeitig einen Wechsel eines saugfähigen Hygieneartikels vornehmen zu können, wodurch beispielsweise ein Wundliegen eines Patienten wirksam verhindert wird. Weiterhin ist durch die Datenbank und den Zugriff auf diese über ein Internetportal sichergestellt, dass von jedem (internetangeschlossenen) Punkt der Welt auf die Informationen, die der Sensor liefert und die sich daraus abzuleitenden Handlungsempfehlungen jederzeit zugegriffen werden kann. Das erhöht den Anwendungsbereich und ist notwendige Voraussetzung, um Management-Prozesse im Pflegebereich prozessual unterstützen zu können. Das Internetportal ist somit als Managementinformationssystem zu klassifizieren.

Erfindungsgemäß ist vorgesehen, dass die Datenübertragung zwischen dem Sender dem Empfänger mittels Bluetooth® hoher Reichweite ("Bluetooth® Long Range") oder über NB-loT (Narrow Band Internet of Things) oder Long Range IoT erfolgt.

Ein hierfür geeignetes Bluetooth®-Modul ist das Bluetooth-Modul "BGM111 Blue Gecko" der Firma Silicon Labs. Dieses Bluetooth-Modul liefert bei einem niedrigen Energieverbrauch eine Reichweite von bis zu 200 Metern und ist somit auch in größeren Pflegeheimen zur Echtzeitüberwachung praktikabel anwendbar. Eine alternative Möglichkeit einer sicheren Datenübertragung über größere Entfernungen bietet das NB-IoT (Narrow Band Internet of Things). NB-IoT ist als sogenannte Low Power Wide Area (LPWA) Technologie mit niedrigem Energiebedarf sowie hoher Gebäudedurchdringung und Reichweite eine kostengünstige Lösung für das schmalbandige Internet der Dinge. Aus diesem Grund enthält die Erfindung nicht nur ein Funkmodul für Bluetooth®, sondern auch für NB-IoT und ist in der Lage auf diesem Standard mehrere Übertragungsprotokolle (beispielsweise "LoRa", eine LPWAN-Technologie im 915 MHz-Bereich mit bis zu 15 km Reichweite) zu bedienen. Damit ist sichergestellt, dass auch dann eine Übertragung der Sensordaten erfolgt, wenn eine der beiden Übertragungsmöglichkeiten technisch kurzzeitig ausfällt. Alternativ dazu findet die Übertragung vom Sensor zum Gateway über Bluetooth® statt, während die Verbindung zwischen Gateway und Datenbank über bekannte Infrastrukturen wie WLAN, LAN und in der Folge WAN abgebildet wird.

Die Anzeigevorrichtung ist besonders vorteilhaft mit einem Internetportal gekoppelt oder mit einer App versehen.

Gemäß einer vorteilhaften Weiterbildung der Erfindung sind der Sensor und/oder der Empfänger und/oder die Anzeigevorrichtung und/oder ein zwischen Empfänger und Ausgabevorrichtung angeordneter Rechner zur Erkennung und Auswertung des Feuchtigkeitsverlaufs über der Zeit geeignet und eingerichtet. Durch die Erkennung des Feuchtigkeitsverlaufs über der Zeit ist eine bessere Erkennung des Beladungszustands des saugfähigen Artikels möglich, da das saugfähige Material für eine gleichmäßige Aufnahme von Feuchtigkeit eine gewisse Zeit benötigt und dennoch bei modernen saugfähigen Artikeln an deren Nutzseite unterhalb einer bestimmten Beladungsschwelle kaum ein Nässegefühl entsteht. Somit kann ein unnötiges vorzeitiges Wechseln von saugfähigen Hygieneartikel vermieden werden, wodurch die Pflegekosten insgesamt reduziert werden. Die Detektierung der Temperatur stellt einen weiteren Parameter dar, der die Zuverlässigkeit für die Beurteilung einer Notwendigkeit eines Wechsels eines saugfähigen Hygieneartikels deutlich erhöht, da auch aus dem Verlauf der Temperatur auf die Miktionsmenge geschlossen werden kann. Das System verwaltet die Anzahl der Hygieneartikelwechsel und verrechnet diese mit einem vom Patienten bzw. Kunden initial eingegebenen Anfangsbestand und löst bei einem individuell einstellbaren Mindestbestand die Nachbelieferung aus.

Abgesehen von der Komfortsteigerung für den Patienten/Kunden wird somit die Transparenz im Verbrauch der Hygieneartikel für alle Beteiligten maximal erhöht. In Verbindung mit weiteren verbunden Sensoren lassen noch erheblich mehr Anwendungsfälle kombinieren. So können Blutdruck, Puls, Blutwerte u.v.m. ebenfalls zu Ereignissen führen, die einzeln oder kombiniert eine massiv bessere Betreuung und Pflege des Patienten bedeuten. In der Folge werden die Pflegedienstleistungen ebenfalls deutlich qualitativ aufgewertet.

Gemäß einer weiteren vorteilhaften Weiterbildung der Erfindung ist vorgesehen, dass der Sensor und/oder der Empfänger und/oder die Ausgabevorrichtung und/oder ein zwischen Empfänger und Ausgabevorrichtung angeordneter Rechner zur Erkennung und Auswertung von flüssigen (Urin) und festen Beladungsbestandteilen (Stuhl) des saugfähigen Artikels geeignet und eingerichtet sind, da die spezifischen unterschiedlichen Ausbreitungen von Feuchtigkeit und Temperatur erfasst werden.

Besonders vorteilhaft ist der Sensor zur Messung von chemischen, biologischen und/oder olfaktorischen Bestandteilen und/oder zur Bestimmung von Über- oder Unterkonzentrationen von biologischen Parametern geeignet und ausgebildet. Damit können weitere wichtige Parameter zur Überwachung des gesamten Unterhaltszustands eines mit einem saugfähigen Artikel versehenen Patienten übermittelt werden. Dies können beispielsweise Eiweißkonzentrationen, Harnsäure-Konzentrationen oder Zuckerkonzentrationen sein.

Besonders vorteilhaft wird als weiterer Parameter die Bewegung eines mit einem saugfähigen Hygieneartikel versehenen Patienten überwacht und erkannt, wofür sich der Bewegungssensor "LIS3DH" der Firma STMicroelectronics eignet. Durch die Überwachung der Bewegung kann festgestellt werden, ob ein Umlagern eines Patienten notwendig wird, oder ob dieser selbst seine Position hinreichend oft verändert. Auch hierdurch wird ein Wundliegen von Patienten wirksam verhindert.

Eine weitere vorteilhafte Ausbildung der Erfindung sieht vor, dass der Sensor der mit diesem verbundenen Sender mittels des Empfängers oder der Auswertungsvorrichtung geortet werden kann. Dies erleichtert in größeren Einrichtungen das Auffinden von verwirrten oder dementen Patienten.

Als Einsatzgebiet der Erfindung können insbesondere Pflegeeinrichtungen, Krankenhäuser oder Altenheime, aber auch mobile Pflegedienste, die Patienten in ihrer privaten Umgebung pflegen. Als saugfähige Hygieneartikel kommen Windeln für Babys, Kleinkinder und Erwachsene ebenso in Betracht wie Einmal-Unterlagen in Betten.

Dabei dient das erfindungsgemäße System dazu, eine Vielzahl von saugfähigen Hygieneartikel über ein einziges Endgerät eines Pflegers gleichzeitig zu überwachen. Die Daten oder die Ergebnisse zentral ausgewerteter Daten aller Sensoren kommen dabei auf einem Gateway oder dem Smartphone des Pflegers zur Anzeige, sodass der Pfleger über die Daten aller Patienten verfügt und zeitnah reagieren kann. Zwischen Sensoren und Anzeigegeräten gibt es eine n zu m Beziehung, d.h. beides funktioniert mit einer Vielzahl von überwachten saugfähigen Hygieneartikel und mehreren Überwachungspersonen im selben System.

Die App kann auf einem oder mehreren empfangenden Endgeräten zeitgleich mehrere Sensoren personalisiert anzeigen, sodass ein Pfleger den Überblick über alle seine Patienten hat und genau erkennen kann, bei welchem Handlungsbedarf besteht. Urin und Stuhlgang können getrennt erkannt und angezeigt werden.

Bei einer parallelen Übertragung desselben Signals an die Endgeräte mehrerer Pfleger ermöglicht die Software demjenigen Pfleger, der sich eines Patienten annimmt, von dem ein Signal eingegangen ist, auch die anderen empfangenden Pfleger per Funksignal darüber zu informieren, dass er diesen Auftrag übernommen hat. Zweckmäßigerweise wird dann bei den anderen Pflegern dieser Auftrag gelöscht oder aus der aktuellen Bearbeitungsliste herausgenommen. Der von einem Pfleger übernommene Auftrag wird zweckmäßigerweise auch automatisch in ein Pflege-Dokumentationssystem für den jeweiligen Patienten und/oder den bearbeitenden Pfleger übernommen.

Nachfolgend werden Ausführungsbeispiele der Vorrichtung unter Bezugnahme auf die Zeichnungen näher erläutert. Es zeigt:
- Fig. 1: eine schematische Darstellung eines Systems am Beispiel eines mit einem Sensor und einem Sender versehenen saugfähigen Artikels, der über einen Empfänger mit wenigstens einem Endgerät in Verbindung steht, und
- Fig. 2: eine schematische Darstellung eines Systems mit mehreren mit jeweils einem Sensor und einem Sender versehenen saugfähigen Artikeln, die über einen Rechner mit mehreren Endgeräten in Verbindung stehen.

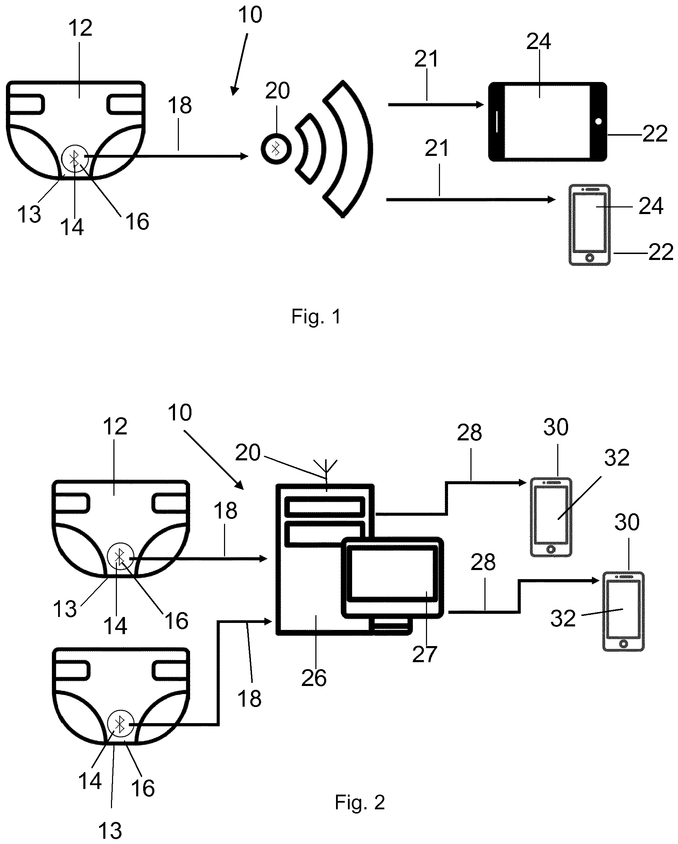

In Figur 1 ein erfindungsgemäßes System 10 dargestellt mit einem als Windel ausgebildeten Hygieneartikel 12, an dem ein Sensor 14 und ein mit diesem baulich zu einer Einheit verbundener Sender 16 lösbar befestigt ist.

Beim Sensor 14 handelt es sich insbesondere z.B. um einen kombinierten Feuchtigkeits- und Temperatursensor "SI7021-A20" der Firma Silicon Labs. Dieser Sensor 14 ist bevorzugt an einer Stelle des saugfähigen Hygieneartikels 12 angeordnet, an der besonders einfach aufgenommene Feuchtigkeit oder feste Beladungsbestandteilen feststellbar sind ― bei einer Windel 12 ist dies beispielsweise der im Schritt des Benutzers angeordnete schmalere Steg 13.

Beim Sender 16 handelt es sich beispielsweise beispielweise um ein Bluetooth®-Modul "BGM111 Blue Gecko" der Firma Silicon Labs. Dieses Bluetooth®-Modul liefert bei einem niedrigen Energieverbrauch eine Reichweite von bis zu 200 Metern und ist somit auch in größeren Pflegeheimen zur Echtzeitüberwachung des Beladungszustands des saugfähigen Hygieneartikels 12 praktikabel anwendbar. Der Sender 16 ist über eine Funk-Übertragungsstrecke 18 mit einem Empfänger 20 verbunden.

Eine alternative Möglichkeit einer sicheren Datenübertragung eines Senders 16 über größere Entfernungen zu einem Empfänger 20 bietet das NB-IoT (Narrow Band Internet of Things). NB-IoT ist als sogenannte Low Power Wide Area (LPWA) Technologie mit niedrigem Energiebedarf sowie hoher Gebäudedurchdringung und Reichweite eine kostengünstige Lösung für das schmalbandige Internet der Dinge.

Der Empfänger 20 ist seinerseits über eine Übertragungsstrecke 21 mit wenigstens einem Endgerät 22 verbunden, dass über eine Anzeigevorrichtung 24 verfügt. Als Beispiel für derartige Endgeräte sind in Figur 1 ein Tablet und ein Smartphone dargestellt. Der Empfänger 20 kann dabei in einer bevorzugten Ausführungsform in das jeweilige Endgerät 22 integriert sein. Auf dem Endgerät 22 ist eine App oder ein ähnliches Programm installiert, das zum Empfang der Signale des Senders 16 und zu einer Darstellung der Auswertung dieser Signale bzw. der vom Sensor 14 gelieferten Messwerte auf der Anzeigevorrichtung 24 geeignet ist. Durch Zuordnung des Sensors 14 bzw. des Senders 16 zu einem bestimmten mit dem saugfähigen Hygieneartikel 12 ausgestatteten Patienten kann ein die Anzeigevorrichtung 24 auswertender Pfleger auch über eine größere Entfernung leicht feststellen, ob ein Wechsel des saugfähigen Hygieneartikels 12 kurzfristig oder mittelfristig erforderlich ist. Bei einem Wechsel des saugfähigen Hygieneartikels 12 werden Sensor 14 und Sender 16, die eine Baueinheit bilden, vom verbrauchten saugfähigen Hygieneartikel 12 abgenommen und am neuen saugfähigen Hygieneartikel 12 lösbar befestigt.

Besonders nützlich ist es, wenn insbesondere bei dauernd liegenden Patienten der Sensor 14 zusätzlich mit einem Sensor zur Detektierung einer Lageveränderung ausgestattet ist. Hier kann dem Pflegepersonal auf der Anzeigevorrichtung 24 zusätzlich angezeigt werden, ob ein Umlagern des Patienten erforderlich ist, um dessen Wundliegen zu verhindern.

Besonders vorteilhaft ist der Sensor 14 zusätzlich zur Messung von chemischen, biologischen und/oder olfaktorischen Bestandteilen und/oder zur Bestimmung von Über- oder Unterkonzentrationen von biologischen Parametern geeignet und ausgebildet. Damit können weitere wichtige Parameter zur Überwachung des gesamten Gesundheitszustands eines mit einem saugfähigen Artikel versehenen Patienten übermittelt werden. Dies können beispielsweise Eiweißkonzentrationen, Harnsäure-Konzentrationen oder Zuckerkonzentrationen sein, die dem Pfleger bei Überschreiten oder unterschreiten einstellbarer Grenzwerte auf der Anzeigevorrichtung 24 die Notwendigkeit eines Eingreifens signalisieren.

In Figur 2 sind exemplarisch zwei saugfähige Hygieneartikel 12 als Beispiel für eine Vielzahl derartiger saugfähige Hygieneartikel 12 in einer Pflegeeinrichtung dargestellt. Die saugfähigen Hygieneartikel 12 sind jeweils mit einem Sensor 14 und einem Sender 16 versehen, die eine Baueinheit bilden.

Die Sender 16 stehen über eine Übertragungsstrecke 18 mit einem Empfänger 20 in drahtloser Funkverbindung, der mit einem Rechner 26 verbunden ist. Der Rechner 26 ist mit einer Anzeigevorrichtung 27 verbunden oder ausgestattet und steht über eine Übertragungsstrecke 28 mit mehreren Endgeräten 30 in einer drahtlosen Funkverbindung. Die Endgeräte 30 sind jeweils mit einer Anzeigevorrichtung 32 ausgestattet. Im gezeigten Ausführungsbeispiel sind die Endgeräte 30 als Smartphones und die Anzeigevorrichtung an 32 als deren Display ausgebildet.

Der Rechner 26 ist mit einem Programm ausgestattet, dass die Signale mehrerer Sender 16 empfängt und jeweils eindeutig einem mit einem saugfähigen Hygieneartikel 12 ausgestatteten Patienten zuordnet. Der Rechner 26 befindet sich beispielsweise in der Zentrale einer Pflegestation eines Krankenhauses, eines Altenheims oder bei einem mobilen Pflegedienst. Die Endgeräte 30 werden von verschiedenen Pflegern mitgeführt. Der Rechner 26 steht bevorzugt in bidirektionaler Datenverbindung über drahtlose Funkübertragungsstrecken 28 mit den mehreren Endgeräten 30. Der Rechner 26 kann dabei über das Programm sowohl die Position der Endgeräte 30 als auch die Position der saugfähigen Hygieneartikel 12 bzw. deren Sender 16 empfangen.

Sobald ein Sensor 14 an einem saugfähigen Hygieneartikel 12 einen Messwert detektiert und über den ihm zugeordneten Sender 16 an den Rechner 26 übermittelt, wird bei Über-oder Unterschreiten wenigstens eines in der Software des Rechners 26 einstellbaren, für den jeweiligen Patienten individuellen Schwellwerts ermittelt, welches Endgerät 30 sich in der Nähe des betreffenden saugfähigen Hygieneartikels 12 befindet und unter Berücksichtigung der momentanen Auftragssituation für den jeweiligen mit dem Endgerät 30 ausgestatteten Pfleger ein neuer Arbeitsauftrag für diesen auf dem Endgerät 30 generiert und über die Anzeigevorrichtung 32 ausgegeben. Der Pfleger kann diesen Auftrag über das Endgerät 30 quittieren oder bei einer temporären Überlastung an den zentralen Rechner 26 zur Weitergabe an ein benachbartes Endgerät 30 zurücksenden.

Zur Garantierung von Datensicherheit und Datenschutz werden die einzelnen Daten zwischen dem wenigstens einen Sender 16 und/oder dem wenigstens einen Empfänger 20 und/oder dem wenigstens einen Rechner 26 und/oder dem wenigstens einen Endgerät 22; 30 bevorzugt verschlüsselt übertragen. Eine unverschlüsselte Übertragung ist ebenfalls Bestandteil des Systems.

### Bezugszeichenliste

- 10: System
- 12: Hygieneartikel (Windel)
- 13: Steg (im Schritt von 12)
- 14: Sensor
- 16: Sender
- 18: Übertragungsstrecke (von 16 auf 20)
- 20: Empfänger
- 21: Übertragungsstrecke (von 20 auf 22)
- 22: Endgerät
- 24: Anzeigevorrichtung
- 26: Rechner
- 27: Anzeigevorrichtung
- 28: Übertragungsstrecke (von 16 auf 26)

- 30: (weiteres) Endgerät
- 32: Anzeigevorrichtung (von 30)

## Patentansprüche

1. System (10) zur Überwachung des Beladungszustands von saugfähigen Hygieneartikeln (12) und zur Auswertung der dabei gewonnenen Messwerte, mit einem an einem saugfähigen Hygieneartikel (12) angeordneten Sensor (14) zur Erzeugung eines Signals in Abhängigkeit von der Feuchte des saugfähigen Hygieneartikels (12),
mit wenigstens einem Sender (16) zur Übertragung des Signals an wenigstens einen Empfänger (20), wobei die Datenübertragung zwischen Sender (16) und Empfänger (20) kabellos in Echtzeit mit einer Reichweite erfolgt, die die typische Reichweite einer einfachen Bluetooth®-Verbindung übersteigt, und wobei der Empfänger (20) das Signal an wenigstens ein als Frontend dienendes, einem Pflegepersonal zugeordnetes Endgerät (22; 30) mit einer Anzeigevorrichtung (24; 32) übermittelt,
wobei ein einziger Sensor (14) außer zur Messung der Feuchtigkeit auch zur Messung der Temperatur des saugfähigen Hygieneartikels (12) eingerichtet ist und mit dem Sender (16) eine bauliche Einheit bildet und wobei der Sensor (14) wiederverwendbar an dem Hygieneartikel (12) befestigbar ist, wobei die Datenübertragung zwischen Sender (16) und Empfänger (20 mittels Bluetooth® hoher Reichweite (Long range) oder über NB-IoT (Narrow Band Internet of Things) oder Long Range IoT erfolgt, und wobei der Sensor (14) außen an dem saugfähigen Hygieneartikel (12) befestigt ist.

2. System (10) nach Anspruch 1, **dadurch gekennzeichnet, dass** das Endgerät (22; 30) für einen Empfang von Signalen mehrerer, an unterschiedlichen Orten oder Nutzern angeordneten Hygieneartikeln (12) geeignet und eingerichtet ist.

3. System (10) nach einem der vorhergehenden Ansprüche, **dadurch gekennzeichnet, dass** das Endgerät (22; 30) und/oder ein zwischen Empfänger (20) und dem Endgerät (22; 30) angeordneter Rechner (26) mit einem Internetportal, mit einer Software oder einer App versehen ist.

4. System (10) nach einem der vorhergehenden Ansprüche, **dadurch gekennzeichnet, dass** der Empfänger (20) und/oder das Endgerät (22; 30) (24) und/oder ein zwischen Empfänger (20) und Endgerät (22; 30) angeordneter Rechner (26) zur Erkennung und Auswertung eines Feuchtigkeitsverlaufs über der Zeit des saugfähigen Hygieneartikels (12) geeignet und eingerichtet ist.

5. System (10) nach einem der vorhergehenden Ansprüche, **dadurch gekennzeichnet, dass** der Empfänger (20) und/oder das Endgerät (22; 30) (24) und/oder ein zwischen Empfänger (20) und Endgerät (22; 30) angeordneter Rechner (26) zur Erkennung und Auswertung von flüssigen und festen Beladungsbestandteilen des saugfähigen Hygieneartikels (12) geeignet und eingerichtet ist.

6. System (10) nach einem der vorhergehenden Ansprüche, **dadurch gekennzeichnet, dass** der Sensor (14) zur Messung von chemischen, biologischen und/oder olfaktorischen Bestandteilen und/oder zur Bestimmung von Über-oder Unterkonzentrationen von biologischen Parametern der Beladung des saugfähigen Hygieneartikels (12) geeignet und ausgebildet ist.

7. System (10) nach einem der vorhergehenden Ansprüche, **dadurch gekennzeichnet, dass** der Empfänger (20) und/oder das Endgerät (22; 30) und/oder der Rechner (26) für einen Empfang der Signale mehrerer Sender (16) an mehreren saugfähigen Hygieneartikeln (12) geeignet und eingerichtet sind.

8. System (10) nach einem der vorhergehenden Ansprüche, **dadurch gekennzeichnet, dass** die Signale mehrerer Sender (16) an mehreren saugfähigen Hygieneartikeln (12) von mehreren Empfängern (20) und/oder Endgeräten (22; 30) und/oder Rechnern (26) empfangbar sind.

9. System (10) nach einem der vorhergehenden Ansprüche, **dadurch gekennzeichnet, dass** zur Garantierung von Datensicherheit und Datenschutz die einzelnen Daten zwischen dem wenigstens einen Sender (16) und/oder dem wenigstens einen Empfänger (20) und/oder dem wenigstens einen Rechner (26) und/oder dem wenigstens einen Endgerät (22; 30) verschlüsselt oder unverschlüsselt übertragen werden.

## Claims

1. System (10) for monitoring the loading state of absorbent hygiene articles (12) and for evaluating the measured values obtained in the process, having a sensor (14), arranged on an absorbent hygiene article (12), for generating a signal on the basis of the moisture in the absorbent hygiene article (12),
having at least one transmitter (16) for transmitting the signal to at least one receiver (20), wherein the data transmission between the transmitter (16) and the receiver (20) takes place wirelessly in real time with a range that exceeds the typical range of a simple Bluetooth® connection, and wherein the receiver (20) conveys the signal to at least one terminal (22; 30), serving as a front end and associated with care personnel, having a display apparatus (24; 32),
wherein a single sensor (14) is designed not only to measure the moisture but also to measure the temperature of the absorbent hygiene article (12) and forms a physical unit with the transmitter (16), and wherein the sensor (14) is able to be reusably mounted on the hygiene article (12), wherein the data transmission between the transmitter (16) and the receiver (20) takes place using long-range Bluetooth® or by way of NB-IoT (narrowband Internet of Things) or long-range IoT, and wherein the sensor (14) is mounted on the outside of the absorbent hygiene article (12).

2. System (10) according to Claim 1, **characterized in that** the terminal (22; 30) is suitable and designed for receiving signals from multiple hygiene articles (12) arranged at different locations or on different users.

3. System (10) according to either of the preceding claims, **characterized in that** the terminal (22; 30) and/or a computer (26) arranged between the receiver (20) and the terminal (22; 30) is provided with an Internet portal, a piece of software or an app.

4. System (10) according to one of the preceding claims, **characterized in that** the receiver (20) and/or the terminal (22; 30) (24) and/or a computer (26) arranged between the receiver (20) and the terminal (22; 30) is suitable and designed for detecting and evaluating a moisture characteristic over time for the absorbent hygiene article (12).

5. System (10) according to one of the preceding claims, **characterized in that** the receiver (20) and/or the terminal (22; 30) (24) and/or a computer (26) arranged between the receiver (20) and the terminal (22; 30) is suitable and designed for detecting and evaluating liquid and solid loading components of the absorbent hygiene article (12).

6. System (10) according to one of the preceding claims, **characterized in that** the sensor (14) is suitable and designed for measuring chemical, biological and/or olfactory components and/or for determining over- or underconcentrations of biological parameters of the loading of the absorbent hygiene article (12).

7. System (10) according to one of the preceding claims, **characterized in that** the receiver (20) and/or the terminal (22; 30) and/or the computer (26) are suitable and designed for receiving the signals from multiple transmitters (16) on multiple absorbent hygiene articles (12).

8. System (10) according to one of the preceding claims, **characterized in that** the signals from multiple transmitters (16) on multiple absorbent hygiene articles (12) are receivable by multiple receivers (20) and/or terminals (22; 30) and/or computers (26).

9. System (10) according to one of the preceding claims, **characterized in that** data security and data protection are guaranteed by virtue of the individual data being transmitted between the at least one transmitter (16) and/or the at least one receiver (20) and/or the at least one computer (26) and/or the at least one terminal (22; 30) in encrypted or unencrypted form.

## Revendications

1. Système (10) de surveillance de l'état de charge d'articles d'hygiène absorbants (12) et d'évaluation des valeurs mesurées obtenues au cours du processus, ledit système comprenant
un capteur (14) disposé sur un article d'hygiène absorbant (12) et destiné à générer un signal en fonction de la teneur en humidité de l'article d'hygiène absorbant (12),
au moins un émetteur (16) destiné à transmettre le signal à au moins un récepteur (20), la transmission de données entre l'émetteur (16) et le récepteur (20) étant effectuée sans fil en temps réel avec une portée qui dépasse la portée typique d'une simple connexion Bluetooth®, et le récepteur (20) transmettant le signal à au moins un terminal (22 ; 30) qui sert d'extrémité frontale, qui est associée à un personnel soignant et qui comporte un dispositif d'affichage (24 ; 32),
un seul capteur (14) étant installé non seulement pour mesurer l'humidité mais également pour mesurer la température de l'article d'hygiène absorbant (12) et formant avec l'émetteur (16) une unité structurelle et le capteur (14) pouvant être fixé de manière réutilisable à l'article d'hygiène (12), la transmission de données entre l'émetteur (16) et le récepteur (20) étant effectuée au moyen de Bluetooth® longue portée (Long range) ou NB-loT (Narrow Band Internet of Things) ou Long Range loT, et le capteur (14) étant fixé à l'extérieur de l'article d'hygiène absorbant (12).

2. Système (10) selon la revendication 1, **caractérisé en ce que** le terminal (22 ; 30) est adapté et conçu pour recevoir des signaux d'une pluralité d'articles d'hygiène (12) disposés à différents endroits ou sur différents utilisateurs.

3. Système (10) selon l'une des revendications précédentes, **caractérisé en ce que** le terminal (22 ; 30) et/ou un ordinateur (26) disposé entre le récepteur (20) et le terminal (22 ; 30) est pourvu d'une connexion Internet portail, d'un logiciel ou d'une application.

4. Système (10) selon l'une des revendications précédentes, **caractérisé en ce que** le récepteur (20) et/ou le terminal (22 ; 30) (24) et/ou un ordinateur (26) disposé entre le récepteur (20) et le terminal (22 ; 30) est adapté et conçu pour détecter et évaluer une variation dans le temps de l'humidité de l'article d'hygiène absorbant (12).

5. Système (10) selon l'une des revendications précédentes, **caractérisé en ce que** le récepteur (20) et/ou le terminal (22 ; 30) (24) et/ou un ordinateur (26) disposé entre le récepteur (20) et le terminal (22 ; 30) est adapté et conçu pour détecter et évaluer des composants de charge liquides et solides de l'article d'hygiène absorbant (12).

6. Système (10) selon l'une des revendications précédentes, **caractérisé en ce que** le capteur (14) est adapté et conçu pour mesurer des composants chimiques, biologiques et/ou olfactifs et/ou déterminer des sur- ou sous-concentrations de paramètres biologiques de la charge de l'article d'hygiène absorbant (12).

7. Système (10) selon l'une des revendications précédentes, **caractérisé en ce que** le récepteur (20) et/ou le terminal (22 ; 30) et/ou l'ordinateur (26) sont adaptés et conçus pour recevoir les signaux de plusieurs émetteurs (16) de plusieurs articles d'hygiène absorbants (12) .

8. Système (10) selon l'une des revendications précédentes, **caractérisé en ce que** les signaux de plusieurs émetteurs (16) de plusieurs articles d'hygiène absorbants (12) peuvent être reçus par plusieurs récepteurs (20) et/ou terminaux (22 ; 30) et/ou ordinateurs (26).

9. Système (10) selon l'une des revendications précédentes, **caractérisé en ce que**, pour garantir la sécurité et la protection des données, les données individuelles sont transmises sous forme cryptée ou non cryptée entre l'au moins un émetteur (16) et/ou l'au moins un récepteur (20) et/ou l'au moins un ordinateur (26) et/ou l'au moins un terminal (22 ; 30).
